(19) Europäisches Patentamt
European Patent Office
Office européen des brevets

(11) **EP 2 089 068 B1**

(12) **EUROPEAN PATENT SPECIFICATION**

(45) Date of publication and mention
of the grant of the patent:
**01.09.2010 Bulletin 2010/35**

(51) Int Cl.:
*A61L 9/14* *(2006.01)*    *B65D 83/16* *(2006.01)*

(21) Application number: **07824466.2**

(22) Date of filing: **07.11.2007**

(86) International application number:
**PCT/GB2007/004231**

(87) International publication number:
**WO 2008/056131 (15.05.2008 Gazette 2008/20)**

(54) **MOTION-SENSING AIR FRESHENER OF THE SPRAY-TYPE**

LUFTERFRISCHER VOM SPRAYTYP MIT BEWEGUNGSMELDER

PURIFICATEUR D'AIR À PULVÉRISATION AVEC DÉTECTION DE MOUVEMENT

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **07.11.2006 GB 0622088**

(43) Date of publication of application:
**19.08.2009 Bulletin 2009/34**

(73) Proprietor: **Reckitt-Benckiser (UK) Limited**
**103-105 Bath Road**
**Slough**
**Berkshire SL3 3UH (GB)**

(72) Inventors:
• **BUTLER, Martin**
**Hull HU8 7DS (GB)**
• **CHAN, Dennis**
**Hull HU8 7DS (GB)**

• **JIN, Wu**
**Hull HU8 7DS (GB)**
• **JONES, Chris**
**Montvale, NJ 07645 (US)**
• **RYMER, Shaun**
**Hull HU8 7DS (GB)**

(74) Representative: **Carlin, Robert George et al**
**Reckitt Benckiser**
**Corporate Services Limited**
**Legal Department - Patents Group**
**Dansom Lane**
**Hull**
**HU8 7DS (GB)**

(56) References cited:
**WO-A-2006/044416    WO-A-2006/084317**
**US-A1- 2005 185 392    US-A1- 2005 201 944**

**Description**

The present invention relates to a device and method for spraying a fluid and particularly, but not exclusively, for spraying fluids such as fragrances, deodorizing fluids and/or pest control materials or the like.

The use of devices to spray fluids such as fragrances into the surrounding environment, such as a room, are well known. For instance automatic spraying devices are generally arranged to spray fluid at regular intervals. Where known automatic air fresheners are concerned, the routine spraying of fragrance can result in phenomenon called fragrance habituation. Habituation is a phenomenon where the benefit of further sprays of fragrance can no longer be appreciated by a user as they have become used to the presence of a residual quantity of the fragrance. A device, which attempts to prevent habituation is described in WO 2006/044416 A2.

A further drawback of known devices is that they are ill equipped to regulate their activity in response to the surrounding environment.

It is an object of the present invention to address the above mentioned concerns and disadvantages.

The invention is defined by the claims.

Preferably the relationship between $t_1$ and $t_2$ is defined by the following formula:

$$t_2 = t_1/x$$

where x = 1.1 to 10

More preferably x = 1.5 to 8, and most preferably x = 2 to 5, and ideally x = 2.

$T_1$ may have a range of 2-120 minutes, and preferably a range of 5-60 minutes, and most preferably a range of 9-36 minutes. The device may be provided with a selection of predefined time intervals for $t_1$. Alternatively, a user may be able to select any value of $t_1$ via inputting such a value into the device by any suitable means, such as by a data entry means or the like.

The device of the present invention may be advantageous because the device is adapted to be responsive to the conditions of the environment surrounding it. In particular, when the device senses motion in the surrounding vicinity it is operable to increase the frequency at which the actuation means actuates thus, in use, increases the quantity of fluid sprayed from a container loaded into the device. The increase in the quantity of fluid sprayed may be capable of minimising or eradicating the habituation phenomenon.

Additionally, by virtue of the device only causing the actuation of the actuation means on detection of subsequent motion in accordance with time interval $t_2$, the device may advantageously possess a degree of self regulation to prevent the device, in use, from spraying a fluid every time motion is detected. This may also prevent the environment surrounding the device from becoming saturated with sprayed fluid.

The device may be operable such that only a maximum number of actuations by the actuation means are permitted within a predefined time period. For instance, the relationship between $t_1$ and $t_2$ defined by the formula:

$t_2 = t_1/x$ , where x = 1.1 to 10, is further subject to the requirement that not more than 9 actuations may occur during a time interval equal to $t_1$, and preferably not more than 7 actuations, and even more preferably not more than 4 actuations.

The motion sensor means may be operable such that after motion has been detected in the surrounding vicinity, the device does is not operable for a period of time, and thus does not consume any power. Preferably after the motion sensor means has detected motion it is not operable for a period of time $\leq t_2$. Even more preferably after the motion sensor means has detected motion it is not operable for a period of time $\leq t_2/2$.

The motion sensor means may be provided in the form of at least one of: an infrared (IR) sensor; a laser sensor; and a sound sensor.

The IR sensor, which is preferably a passive IR sensor, may be operable to detect radiation in the infrared spectrum, thus be capable of detecting the presence of a person or animal within the vicinity of the device. The laser sensor may be operable to emit one or more laser beams and be adapted to detect when an object breaks the one or more beams by moving across the beam(s), thus indicating the presence of a person or an animal within the vicinity of the device. The sound sensor may be operable to detect sound within the vicinity of the device and, preferably, once the detected sound exceeds a predefined level this is indicative of movement within the vicinity of the device.

The motion sensor means may be provided by at least two of: an infrared (IR) sensor; a laser sensor; a sound sensor. Preferably the motion sensor means is provided by an infrared (IR) sensor and a laser sensor and a sound sensor.

Motion within the vicinity of a device according to the present invention may be defined as one or more 'motion events' within the vicinity of the device. The motion sensor means may be operable to detect each motion event within the vicinity of the device and communicate each event to the controller. Alternatively or additionally, the motion sensor means may only communicate the detection of a motion event to the controller once a predefined number of motion events have been detected. As a further alternative or additional arrangement, the controller may only communicate with the actuation means to cause the actuation thereof once a predefined number of motion events has been communicated to the controller by the motion sensor means.

The number of predefined motion events that may be required in order to cause the actuation of the actuation means may be fixed or may be selectable by a user. The possibility for a user to select the number of predetermined motion events required to trigger an actuation means may be advantageous as a user can modify the number based on the location of the device and the user's requirements of the device.

The controller may be provided as a discreet component of the device. Alternatively the controller may be integral with or a part of the motion sensor means. As a further alternative, the controller may be integral with or a part of the actuation means.

The housing is preferably shaped such that it is capable of substantially completely surrounding a container of fluid. The housing preferably has an aperture to allow, in use, the spraying of the fluid from the container therethrough. Preferably the motion sensor means is located on the device in a position remote from the aperture. Even more preferably the motion sensor means is located on an upper portion of the device spaced away from and above the aperture. This latter arrangement of the motion sensor means may be advantageous as being located above the aperture may prevent sprayed fluid erroneously coming into contact with the sensor means, thus prevent the sensor means from becoming clouded with fluid and less sensitive to detecting motion in the vicinity of the device.

The device may be operable in a normal mode or a detection mode; wherein in normal mode the actuation means may be operable, in use, to actuate at the time interval of $t_1$ and wherein in detection mode the motion sensor means may be operable, in use, to detect motion in the vicinity of the device and communicate any detection to the controller which causes the actuation means to actuate; and wherein subsequent detection of motion by the motion sensor means may be communicated to the controller which causes the actuation means to actuate at a time interval of $t_2$; and wherein $t_2 \leq t_1$.

The device may be switchable between the normal mode and the detection mode. The device may be manually or automatically switchable between the normal mode and the detection mode. Automatic switching between normal mode and detection mode may be controlled by a timing mechanism and/or a sensor operably connected to the controller, such as a light sensor and/or sound detection means.

The incorporation of a timing mechanism to effect the switching of the device between a normal mode and a detection mode may be advantageous as a user can select when the detection mode may be operable, thus, providing a user with a greater level of control as to when the actuation frequency may be increased in response to motion being detected. This level of control will also permit a user to conserve the power consumption of the device by controlling the amount of time the device is in the detection mode, thus, controlling when the motion sensor means may consume power.

Furthermore, the incorporation of a sensor to effect the switching of the device between a normal mode and a detection mode may be advantageous as a user can allow the device to automatically cause the switching between a normal mode and a detection mode providing a user with a greater level of control as to when the actuation frequency may be increased in response to motion being detected. For instance, a light sensor may be used to only allow the switching when light is detected such that motion at night does not cause the actuation of the actuation means. Whereas a sound detection means may permit the switching into the detection mode only when sound is detected, thus, preventing the motion sensor means from consuming power until sound is detected, the sound possibly being indicative of the environment around the device being used.

The device may be provided with an indicator wherein said indicator is operable to indicate to a user what function the device is currently performing. The indicator may be operable to provide a visual indication and/or provide an audible indication.

Preferably the indicator is configured to provide a visual indication by emitting light from one or more light sources, preferably one or more LEDs.

The one or more light sources may be adapted to emit a different colour of light to indicate the current function the device is performing. Additionally or alternatively, the one or more light sources may blink or flash to indicate the current function the device is performing.

Alternatively or additionally, the device may be operable to visually indicate the function currently being performed by the device via a screen. The screen may be an LCD screen that is adapted to provide a message to a user, for instance such messages could include "ON", "SENSING", "MOTION DETECTED", "RESTING", "NORMAL MODE", "DETECTION MODE", "OFF".

The device may be provided with a boost mechanism. The boost mechanism may be linked to a user operated switch or button or the like. On operating the boost mechanism the actuation means may actuate, and this actuation may occur regardless of the current mode of operation of the device. In effect, the boost mechanism may provide the user with a control to override the operation of the device for a single actuation.

The device may be power by mains-supplied electricity and/or be battery powered and/or be powered by solar cells located on the device. Most preferably the device is battery powered.

The container of fluid for use with the device of the present invention is preferably an aerosol can of fluid, and preferably a metered dose aerosol can of fluid.

According to a second aspect of the present invention there is provided therefore a method of spraying a fluid from a fluid container, the method comprising the steps of:

loading a container of fluid into a device according to the first aspect of the present invention;
placing the device in an operational mode wherein the motion sensor means is capable of detecting motion in the vicinity of the device;
and wherein, upon detection of motion by said motion sensor means, the controller causes the actuation means to actuate thus causing the spraying of fluid from the container into the environment surrounding the device.

When the device is first placed in an operational mode the controller may cause the actuation means to actuate substantially immediately or after a short delay, say after 2-20 seconds, to cause a quantity of fluid to be sprayed. Should motion be detected immediately after the fluid has been sprayed the controller may cause the actuation means to actuate again with subsequent actuations taking place at the time interval $t_2$ as prescribed above.

Alternatively the controller may, after the first actuation following being placed in an operational mode and on being informed of detection of motion within the vicinity of the device, delay causing the actuator to actuate until a predetermined interval of time $t_3$ has elapsed. Once time period $t_3$ has elapsed subsequent actuation following the detection of motion would take place at a time interval of $t_2$ as prescribed above. Preferably the predetermined interval of time $t_3 = t_2/y$ , where y = 1.1 to 10.

According to a third aspect of the present invention there is provided therefore a kit of parts for spraying a quantity of fluid, said kit comprising a device in accordance with the first aspect of the present invention, said device being adapted to operate in accordance with the method according to the second aspect of the present invention, and further comprising a container of fluid wherein said container is configured to be loadable into the housing of the device.

Embodiments of the invention will now be described, by way of example only, with reference to the following description and drawings in which:

Fig.1 illustrates a front elevation of the device with a front cover removed; and
Fig 2. illustrates a front elevation of the device with the front cover in place.

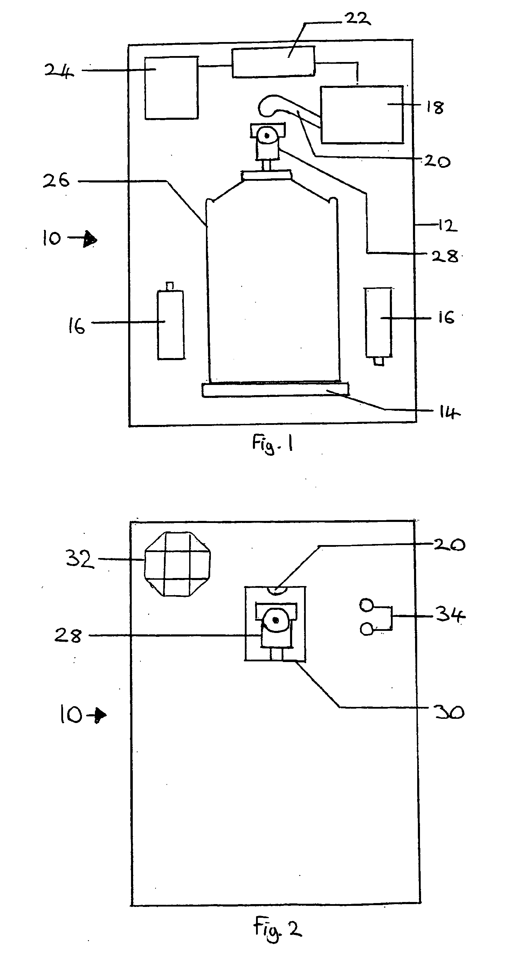

As shown in Fig. 1, the spraying device 10 comprises a housing 12 which supports a platform 14. The platform 14, in turn, is shaped to support and retain a container of fluid 26, such as an aerosol of fluid, when the device is in use. The housing 12 also supports an actuation means 18, a controller 22, a motion sensor means 24 and a power source which in Fig. 1 is depicted as a pair of batteries 16, although more or less batteries may be used.

The device 10 is illustrated with a container of fluid 26 loaded therein and, specifically, Fig. 1 is depicted with an aerosol of fluid loaded therein. The fluid is sprayed from the can an arm 20 connected to the actuation means 18 being moved in a downward direction and into contact with a spray head 28 of the aerosol. The movement of the arm 20 continues until the spray head is depressed and the valve within the aerosol is opened, thus, causing a quantity of fluid to be sprayed therefrom. Preferably the device 10 has a metered dose aerosol loaded therein. A metered dose aerosol being advantageous as a single depression of the spray head will release a predefined quantity of fluid from the aerosol regardless of the duration of time the spray head is depressed. However, a non-metered dose aerosol may be used in the device 10 as could a non-pressurised container possessing a pump mechanism to spray the fluid therefrom.

Alternatively, the actuation means 18 could take the form of a valve system, such as a solenoid valve system. Such a solenoid valve system may work together with a pressurised aerosol engaged therewith. Rather than initiate actuation by movement, the solenoid valve would be energised to initiate the release of a quantity of fluid from the aerosol.

Regardless of the specific form of the actuation means 18, the mode of operation of the device and the inter-relation of the components will now be explained in detail.

The device 10 must first be placed in an operational mode. There will be a user-activated switch (not shown) to permit the device to be switched into the operational mode. The device 10 will draw power from the power source which may be mains electric power and/or solar cells mounted on the device, but is depicted in Fig. 1 as a pair of batteries 16.

Initiating the operational mode will cause the immediate spraying of fluid, as described above. Thereafter further sprays of fluid will periodically occur at time intervals of $t_1$. A user controlled switch (not shown) may be provided to allow a user to adjust the value of $t_1$.

However, the motion sensor means 24 may also draw power from the batteries 16, either constantly or periodically, in order to sense for movement in the vicinity of the device 10. If the motion sensor means 24 senses movement it is operable to communicate this information to the controller 22. Once the controller 22 has received this information it is operable to instruct the actuation means 18 to actuate again, thus causing a further spray of fluid. Subsequent detection of motion by the motion sensor means 24 may also be communicated to the controller which will cause the actuation means to actuate at a time interval of $t_2$ after the previous actuation, subject to $t_2 \leq t_1$.

When the device is first placed in an operational mode the controller 22 may cause the actuation means 18 to actuate substantially immediately or after a short delay, say after 2-20 seconds, to cause a quantity of fluid to be sprayed. Should motion be detected immediately after the fluid has been sprayed the controller 22 may cause the actuation means 18 to actuate again with subsequent actuations taking place at the time interval $t_2$.

Alternatively the controller 22 may, after the first actuation following being placed in an operational mode and on being informed of detection of motion within the vicinity of the device, delay causing the actuator 18 to actuate until a predetermined interval of time $t_3$ has elapsed. Once time period $t_3$ has elapsed subsequent actuation following the detection of motion would take place at a time interval of $t_2$.

The device 10 may be switchable between a normal mode and a detection mode, a user controllable switch (not shown) may be provided. In this embodiment the normal mode of operation permits the actuator 18 to actuate at the routine time interval of $t_1$. When the device is switched into detection mode however, the motion sensor means 24 may be operable to detect motion in the vicinity of the device. Should motion be detected, this information is communicated to the controller 22 which causes the actuation means 28 to actuate. Whilst the device remains in detection mode subsequent detection of motion by the motion sensor means 24 is communicated to the controller 22 which causes the actuation means 18 to actuate at a time interval of $t_2$, wherein $t_2 \leq t_1$.

The device may be automatically switchable between the normal mode and the detection mode. Such automatic switching may be controlled by a timing mechanism and/or a sensor operably connected to the controller, such as a light sensor and/or sound detection means. The automatic switching may permit the device to consume less power by only permitting the device to operate in the detection mode of a limited period of time, thus conserving the power consumed by the motion sensor means 24. Such conservation of power being particularly useful where the device is power by batteries 16 and or solar cell(s).

Turning to Fig. 2, the housing may be shaped such that it is capable of substantially completely surrounding a container of fluid 26. The front cover of the housing is illustrated and it can be seen that the front cover includes an aperture 30 therethrough which is in registration with the spray head 28 of the container 26. When the actuation means 18 cause the spraying of the fluid, the fluid exits the housing through the aperture 30 into the environment surrounding the device 10.

The motion sensor means 24 has a lens cover 32, which protrudes from the front of the device 10 to ensure a wide field of view. This may be advantageous where the motion sensor means is a passive infra-red sensor, such as a Panasonic PIR MP motion sensor AMN1 (as manufactured by Panasonic), since the motion would not need to be directly in front of the sensor means to be detected. Similarly, where the motion sensor means 24 is additionally or alternatively provided as a laser sensor or a flickering light sensor, this will also afford such sensors a wide field of view.

The lens 32 is located on, or protrudes through, an upper portion of the front cover of the housing in a position remote from the aperture 30. This ensures that the lens 32 is spaced away from and above the aperture which may prevent sprayed fluid erroneously coming into contact with the lens 32, thus preventing the lens 32 from becoming clouded with fluid over time.

Similarly, where the motion sensor means 24 incorporates, or comprises solely, a sound sensor, a port or aperture or microphone for receiving audio may also be positioned on an upper portion of the front cover of the housing in a position remote from the aperture 30 to prevent the sensor from coming into contact with sprayed fluid which may impair the sensing properties of said sensor.

Fig. 2 further illustrates an indicator 34 which is provided in the form of two LEDs. The LEDs may be operable to provide a visual indication of the function currently being performed by the device. For instance, one LED could indicate when the device is in an operational mode and the other LED could indicate when motion has bee detected.

The indicator 34 may also be provided with an audio component (not shown) wherein this component is capable of giving an audible alert when a particular function is being performed and/or motion has been detected or the like.

Alternatively or additionally, the indicator 34 may be provided with an LCD screen (not shown) where the screen is adapted to provide a message to a user indicating the current functioning of the device 10. For instance such messages could include "ON", "SENSING", "MOTION DETECTED", "RESTING", "NORMAL MODE", "DETECTION MODE", "OFF".

**Claims**

1. A method of spraying a fluid from a spraying device, wherein the spraying device comprises:

   a housing adapted to receive a container of fluid; actuation means configured, in use, for periodic actuation of the container of fluid; motion sensor means; a controller in operable communication with said actuation means and said motion sensor means; and
   wherein the method comprises:

   causing the actuation means to actuate at a time interval of $t_1$;
   detecting motion with the motion sensor means in the vicinity of the device and communicating any detection to the controller and the controller causes the actuation means to actuate;
   when subsequently detecting motion by the motion sensor, communicating this detection to the controller, the controller causes the actuation means to actuate at a time interval of $t_2$ after the previous actuation wherein $t_2 \leq t_1$.

2. The method of spraying according to claim 1, wherein the controller is configured to cause the actuation means to actuate according to the relationship be-

tween $t_1$ and $t_2$ being defined by the following formula: $t_2 = t_1/x$, where x = 1.1 to 10

3. The method of spraying according to claim 1, wherein the controller is configured to cause the actuation means to actuate according to the relationship between $t_1$ and $t_2$ being defined by the following formula: $t_2 = t_1/x$, where x = 1.5 to 8.

4. The method of spraying according to claim 1, wherein the controller is configured to cause the actuation means to actuate according to the relationship between $t_1$ and $t_2$ being defined by the following formula: $t_2 = t_1/x$, where x = 2 to 5, and preferably x = 2.

5. The method of spraying according to any preceding claim, wherein the controller is configured to cause the actuation means not to actuate beyond a maximum number of actuations during a time interval equal to $t_1$.

6. The method of spraying according to any preceding claim, wherein after the motion sensor has communicated detection of motion to the controller and the controller has caused the actuation means to actuate, the controller is further configured to cause the motion sensor to not be operable for a period of time $\leq t_2$, and preferably for a period of time $\leq t_2/2$.

7. The method of spraying according to any preceding claim, wherein the motion sensor means only communicates the detection of a motion event to the controller once a predefined number of motion events have been detected.

8. The method of spraying according to any of claims 1-6, wherein the controller only communicates with the actuation means to cause the actuation thereof once a predefined number of motion events have been communicated to the controller by the motion sensor means.

9. The method of spraying according to any preceding claim, wherein the controller is configured to be operable in a normal mode or in a detection mode; wherein in normal mode the controller is operable to cause the actuation means to actuate at the time interval of $t_1$; and wherein in detection mode the motion sensor means is operable to detect motion in the vicinity of the device and communicate any detection to the controller which causes the actuation means to actuate and wherein subsequent detection of motion by the motion sensor means is communicated to the controller which causes the actuation means to actuate at a time interval of $t_2$ wherein $t_2 \leq t_1$.

10. The method of spraying according to claim 9, wherein a user controls whether the device is operating in the normal mode or the detection mode.

11. The method of spraying according to claim 9, wherein the operation of the device in normal mode or the detection mode is controlled by a timing mechanism and/or a sensor operably connected to the controller.

12. A method of spraying a fluid from a fluid container, the method comprising the steps of:

loading a container of fluid into a device operable in accordance with the method according to any preceding claim; placing the device in an operational mode wherein the motion sensor means is capable of detecting motion in the vicinity of the device; and wherein, upon detection of motion by said motion sensor means, the controller causes the actuation means to actuate in accordance with the method of the preceding claims.

13. A spraying device comprising:

a housing adapted to receive a container of fluid; actuation means configured, in use, for periodic actuation of the container of fluid; motion sensor means; a controller in operable communication with said actuation means and said motion sensor means:

wherein the device comprises means for carrying out the method according to any of claims 1-11.

14. A kit of parts for spraying a quantity of fluid, said kit comprising a device in accordance with claim 13, said device being configured to operate in accordance with the method of any of claims 1-11, and further comprising a container of fluid wherein said container is configured to be loadable into the housing of the device.

**Patentansprüche**

1. Verfahren zum Sprühen eines Fluids aus einer Sprühvorrichtung, wobei die Sprühvorrichtung umfasst:

ein Gehäuse, dass so ausgelegt ist, dass es einen Behälter mit Fluid aufnimmt; Betätigungsmittel, die bei Verwendung zur periodischen Betätigung des Behälters mit Fluid konfiguriert sind; Bewegungssensormittel; eine Steuereinheit in funktionsfähiger Kommunikation mit den Betätigungsmitteln und den Bewegungssensormitteln; und
wobei das Verfahren umfasst:

Bewirken, dass die Betätigungsmittel in einem Zeitintervall von $t_1$ tätig werden;
Erkennen von Bewegung mit den Bewegungssensormitteln in der Nachbarschaft der Vorrichtung und
Kommunizieren jeglicher Erkennung an die Steuereinheit, wobei die Steuereinheit bewirkt, dass die Betätigungsmittel tätig werden;
wobei bei anschließender Erkennung von Bewegung durch den Bewegungssensor und Kommunizieren dieser Erkennung an die Steuereinheit die Steuereinheit bewirkt, dass die Betätigungsmittel in einem Zeitintervall von $t_2$ nach der vorherigen Betätigung tätig werden, wobei $t_2 \leq t_1$.

2. Sprühverfahren nach Anspruch 1, wobei die Steuereinheit so konfiguriert ist, dass sie bewirkt, dass die Betätigungsmittel gemäß der Beziehung zwischen $t_1$ und $t_2$ tätig werden, die durch die folgende Formel definiert ist:

$$t_2 = t_1/x, \text{ wobei } x = 1{,}1 \text{ bis } 10.$$

3. Sprühverfahren nach Anspruch 1, wobei die Steuereinheit so konfiguriert ist, dass sie bewirkt, dass die Betätigungsmittel gemäß der Beziehung zwischen $t_1$ und $t_2$ tätig werden, die durch die folgende Formel definiert ist:

$$t_2 = t_1/x, \text{ wobei } x = 1{,}5 \text{ bis } 8.$$

4. Sprühverfahren nach Anspruch 1, wobei die Steuereinheit so konfiguriert ist, dass sie bewirkt, dass die Betätigungsmittel gemäß der Beziehung zwischen $t_1$ und $t_2$ tätig werden, die durch die folgende Formel definiert ist:

$$t_2 = t_1/x, \text{ wobei } x = 2 \text{ bis } 5 \text{ und vorzugsweise } x = 2.$$

5. Sprühverfahren nach einem der vorhergehenden Ansprüche, wobei die Steuereinheit so konfiguriert ist, dass sie bewirkt, dass die Betätigungsmittel während eines Zeitintervalls, das $t_1$ entspricht, nicht über eine maximale Anzahl von Betätigungen hinaus tätig werden.

6. Sprühverfahren nach einem der vorhergehenden Ansprüche, wobei die Steuereinheit ferner so konfiguriert ist, dass sie, nachdem der Bewegungssensor die Erkennung von Bewegung an die Steuereinheit kommuniziert hat und die Steuereinheit bewirkt hat, dass die Betätigungsmittel tätig werden, bewirkt, dass der Bewegungssensor für eine Zeitdauer $\leq t_2$ und vorzugsweise für eine Zeitdauer $\leq t_2/2$ nicht funktionsfähig ist.

7. Sprühverfahren nach einem der vorhergehenden Ansprüche, wobei das Bewegungssensormittel die Erkennung eines Bewegungsereignisses erst an die Steuereinheit kommuniziert, sobald eine vordefinierte Anzahl von Bewegungsereignissen erkannt wurde.

8. Sprühverfahren nach einem der Ansprüche 1 bis 6, wobei die Steuereinheit mit den Betätigungsmitteln erst kommuniziert, um die Betätigung davon zu bewirken, sobald eine vordefinierte Anzahl von Bewegungsereignissen durch die Bewegungssensormittel an die Steuereinheit kommuniziert wurde.

9. Sprühverfahren nach einem der vorhergehenden Ansprüche, wobei die Steuereinheit so konfiguriert ist, dass sie in einem Normalmodus oder in einem Erkennungsmoduls betrieben werden kann;
wobei die Steuereinheit im Normalmodus so betrieben werden kann, dass sie bewirkt, dass die Betätigungsmittel im Zeitintervall von $t_1$ tätig werden; und
wobei das Bewegungssensormittel im Erkennungsmodus so betrieben werden kann, dass es Bewegung in der Nachbarschaft der Vorrichtung erkennt und jegliche Erkennung an die Steuereinheit kommuniziert, welche bewirkt, dass die Betätigungsmittel tätig werden, und wobei eine anschließende Erkennung von Bewegung durch die Bewegungssensormittel an die Steuereinheit kommuniziert wird, welche bewirkt, dass die Betätigungsmittel in einem Zeitintervall von $t_2$ tätig werden, wobei $t_2 \leq t_1$.

10. Sprühverfahren nach Anspruch 9, wobei ein Benutzer steuert, ob die Vorrichtung im Normalmodus oder im Erkennungsmodus funktioniert.

11. Sprühverfahren nach Anspruch 9, wobei der Betrieb der Vorrichtung im Normalmodus oder im Erkennungsmodus durch einen Zeitsteuerungsmechanismus und/oder einen Sensor, der funktionsfähig mit der Steuereinheit verbunden ist, gesteuert wird.

12. Verfahren zum Sprühen eines Fluids aus einem Fluidbehälter, wobei das Verfahren die folgenden Schritte umfasst:

Laden eines Behälters mit Fluid in eine Vorrichtung, die gemäß dem Verfahren nach einem der vorhergehenden Ansprüche betrieben werden kann;
Versetzen der Vorrichtung in einen Betriebsmodus, in welchem das Bewegungssensormittel zum Erkennen von Bewegung in der Nachbarschaft der Vorrichtung imstande ist;
und wobei bei Erkennung von Bewegung durch die Bewegungssensormittel die Steuereinheit bewirkt, dass die Betätigungsmittel gemäß dem Verfahren der vorhergehenden Ansprüche tätig werden.

**13.** Sprühvorrichtung, umfassend:

ein Gehäuse, das so ausgelegt ist, dass es einen Behälter mit Fluid aufnimmt;
Betätigungsmittel, die bei Verwendung zur periodischen Betätigung des Behälters mit Fluid konfiguriert sind;
Bewegungssensormittel;
eine Steuereinheit in funktionsfähiger Kommunikation mit den Betätigungsmitteln und den Bewegungssensormitteln;
wobei die Vorrichtung Mittel zum Ausführen des Verfahrens nach einem der Ansprüche 1 bis 11 umfasst.

**14.** Kit von Teilen zum Sprühen einer Menge von Fluid, wobei der Kit eine Vorrichtung nach Anspruch 13 umfasst, die Vorrichtung so konfiguriert ist, dass sie gemäß dem Verfahren nach einem der Ansprüche 1 bis 11 funktioniert, und ferner umfassend einen Behälter mit Fluid, wobei der Behälter so konfiguriert ist, dass er in das Gehäuse der Vorrichtung geladen werden kann.

**Revendications**

**1.** Procédé de pulvérisation d'un fluide à partir d'un dispositif de pulvérisation, où le dispositif de pulvérisation comprend :

un logement adapté pour recevoir un contenant de fluide ; un moyen d'actionnement configuré, en utilisation, pour un actionnement périodique du contenant de fluide ; un moyen de capteur de mouvement ; une unité de commande en communication opérationnelle avec ledit moyen d'actionnement et ledit moyen de capteur de mouvement ; et
où le procédé comprend les étapes consistant à :

amener le moyen d'actionnement à actionner à un intervalle de temps de $t_1$ ;
détecter un mouvement avec le moyen de capteur de mouvement en voisinage du dispositif et communiquer toute détection à l'unité de commande, l'unité de commande amenant le moyen d'actionnement à actionner ;
lors de la détection ultérieure d'un mouvement par le capteur de mouvement, communiquer cette détection à l'unité de commande, l'unité de commande amenant le moyen d'actionnement à actionner à un intervalle de temps de $t_2$ après l'actionnement précédent où $t_2 \leq t_1$.

**2.** Procédé de pulvérisation selon la revendication 1, dans lequel l'unité de commande est configurée pour amener le moyen d'actionnement à actionner selon la relation entre $t_1$ et $t_2$ définie par la formule suivante : $t_2 = t_1/x$, où x = 1,1 à 10.

**3.** Procédé de pulvérisation selon la revendication 1, dans lequel l'unité de commande est configurée pour amener le moyen d'actionnement à actionner selon la relation entre $t_1$ et $t_2$ définie par la formule suivante : $t_2 = t_1/x$, où x = 1,5 à 8.

**4.** Procédé de pulvérisation selon la revendication 1, dans lequel l'unité de commande est configurée pour amener le moyen d'actionnement à actionner selon la relation entre $t_1$ et $t_2$ définie par la formule suivante : $t_2 = t_1/x$, où x = 2 à 5 et de préférence x = 2.

**5.** Procédé de pulvérisation selon l'une quelconque des revendications précédentes, dans lequel l'unité de commande est configurée pour amener le moyen d'actionnement à ne pas actionner au-delà d'un nombre maximum d'actionnements pendant un intervalle de temps égal à $t_1$.

**6.** Procédé de pulvérisation selon l'une quelconque des revendications précédentes, dans lequel après que le capteur de mouvement a communiqué une détection de mouvement à l'unité de commande et que l'unité de commande a amené le moyen d'actionnement à actionner, l'unité de commande est en outre configurée pour amener le capteur de mouvement à ne pas être opérationnel pendant une durée $\leq t_2$, et de préférence pendant une durée $\leq t_2/2$.

**7.** Procédé de pulvérisation selon l'une quelconque des revendications précédentes, dans lequel le moyen de capteur de mouvement ne communique la détection d'un évènement de mouvement à l'unité de commande qu'une fois qu'un nombre prédéfini d'évènements de mouvement a été détecté.

**8.** Procédé de pulvérisation selon l'une quelconque des revendications 1 à 6, dans lequel l'unité de commande ne communique avec le moyen d'actionnement pour amener son actionnement qu'une fois qu'un nombre d'évènements de mouvement a été communiqué à l'unité de commande par le moyen de capteur de mouvement.

**9.** Procédé de pulvérisation selon l'une quelconque des revendications précédentes, dans lequel l'unité de commande est configurée pour être opérationnelle dans un mode normal ou dans un mode de détection ;

où dans le mode normal, l'unité de commande est opérationnelle pour amener le moyen d'actionnement à actionner à l'intervalle de temps de $t_1$ ; et

où dans le mode de détection, le moyen de capteur de mouvement est opérationnel pour détecter le mouvement au voisinage du dispositif et communiquer toute détection à l'unité de commande qui amène le moyen d'actionnement à actionner et où une détection ultérieure de mouvement par le moyen de capteur de mouvement est communiquée à l'unité de commande qui amène le moyen d'actionnement à actionner à un intervalle de temps de $t_2$ où $t_2 \leq t_1$.

10. Procédé de pulvérisation selon la revendication 9, dans lequel un utilisateur commande l'état de fonctionnement en mode normal ou en mode de détection du dispositif.

11. Procédé de pulvérisation selon la revendication 9, dans lequel le fonctionnement du dispositif en mode normal ou en mode de détection est commandé par un mécanisme de chronologie et/ou un capteur connecté de façon opérationnelle à l'unité de commande.

12. Procédé de pulvérisation d'un fluide à partir d'un contenant de fluide, le procédé comprenant les étapes consistant à :

charger un contenant de fluide dans un dispositif opérationnel conformément au procédé selon l'une quelconque des revendications précédentes ;

placer le dispositif dans un mode opérationnel où le moyen de capteur de mouvement est capable de détecter un mouvement au voisinage du dispositif ;

et où, lors de la détection de mouvement par ledit moyen de capteur de mouvement, l'unité de commande amène le moyen d'actionnement à actionner conformément au procédé des revendications précédentes.

13. Dispositif de pulvérisation comprenant :

un logement adapté pour recevoir un contenant de fluide ;

un moyen d'actionnement configuré, en utilisation, pour un actionnement périodique du contenant de fluide ;

un moyen de capteur de mouvement ;

une unité de commande en communication opérationnelle avec ledit moyen d'actionnement et ledit moyen de capteur de mouvement ;

dans lequel le dispositif comprend un moyen pour réaliser le procédé selon l'une quelconque des revendications 1 à 11.

14. Nécessaire de pièces destiné à pulvériser une quantité de fluide, ledit nécessaire comprenant un dispositif conforme à la revendication 13, ledit dispositif étant configuré pour fonctionner conformément au procédé selon l'une quelconque des revendications 1 à 11, et comprenant en outre un contenant de fluide où ledit contenant est configuré pour pouvoir être chargé dans le logement du dispositif.

Fig. 1

Fig. 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006044416 A2